# EUROPEAN PATENT APPLICATION

(11) **EP 3 015 068 A1**
(43) Date of publication of application: **04.05.2016**
(21) Application number: 13890324.0
(22) Date of filing: 01.08.2013
(51) Int. Cl.: A61B 8/00, H04N 5/30

(54) **DEVICE AND METHOD FOR ACQUIRING FUSION IMAGE**

(71) Applicant: Sogang University Research Foundation, Mapo-gu, Seoul 121-742 (KR)
(72) Inventor: CHANG, Jin Ho, Seoul 158-774 (KR); SONG, Tai-Kyong, Seoul 110-012 (KR); YOO, Yang Mo, Goyang-si Gyeonggi-do 411-730 (KR); KANG, Jeeun, Seoul 121-140 (KR); WILSON, Brian, Toronto, Ontario M6R 2Y6 (CA); KIM, Kang, Pittsburgh, Pennsylvania 15237 (US); HAN, Seung Hee, Seoul 131-875 (KR)
(74) Representative: V.O.
(86) International application number: PCT/KR2013/006943
(87) International publication number: WO 2015/016403

(57) **Abstract**

Disclosed is a device and method for acquiring a fusion image, which applies an ultrasound signal for an ultrasound image, an optical signal for a photoacoustic image and a fluorescent image to a target, receives an ultrasound signal, a photoacoustic signal and an optical signal from the target, and generates a fusion image including image information with different probing planes with respect to the target by using at least two signals of the received ultrasound signal, the received photoacoustic signal and the received optical signal.

## Description

### [Technical Field]

This disclosure relates to a medical image technique for diagnosis, analysis and treatment, and more particularly, to a probe structure, an imaging device and an imaging method for acquiring a fusion image capable of providing pathologic and anatomical information simultaneously based on various medical image techniques.

### [Background Art]

An ultrasound (US) imaging device is equipment for imaging structure and characteristics of an observation area by applying an ultrasound signal to an observation area in a human body with an ultrasound probe, receiving a returning ultrasound signal reflected by tissues, and extracting information included in the signal. The US imaging device may advantageously obtain an image in real time without any harm to the human body at low costs in comparison to other medical imaging systems such as X-ray, CT, MRI, PET or the like.

A photoacoustic (PA) imaging device applies photon to an observation area in a human body, receives an ultrasound signal directly generated from photons absorbed in tissues, and extracts image information from the signal. This peculiar situation where photons are absorbed in tissues to generate an ultrasound happens since the tissues are heated while absorbing the photons. Thus, if a pulse laser is irradiated to the absorptive tissue structure, the tissue temperature changes, and as a result the tissue structure is expanded. A pressure wave is propagated outwards from the expanded structure, and the pressure wave may be probed using an ultrasound transducer. The photoacoustic image has advantages in that an image may be obtained based on an optical absorption contrast ratio while ensuring resolution to the level of ultrasound, costs are very low in comparison to MRI, and patents are not exposed to ionizing radiation.

A fluorescent (FL) imaging device uses a principle that, cells or bacteria where a fluorescent protein gene is expressed are marked or put into a living body and a light source of a specific wavelength is irradiated thereto, the cells or tissues of the living body or a fluorescent material in the living body absorbs and excites the light irradiated from the outside to emit a light of a specific wavelength, and this light is probed and imaged. As the fluorescent protein gene required for acquiring a fluorescent image, green fluorescent protein (GFP), red fluorescent protein (RFP), blue fluorescent protein (BFP) and yellow fluorescent protein (YEP) or enhanced GFP (EGFP) which is a variety of GFP are widely used, and more diverse fluorescent proteins with increased brightness are being developed. The fluorescent image is generally acquired using a charged coupled device (CCD) camera, which allows rapid acquisition of a fluorescent image, and animals such as guinea pig are not sacrificed.

Such medical diagnosis imaging devices have different observation areas and characteristics, and thus different kinds of devices should be applied to a single observation area depending on purpose and situation. In addition, for more accurate diagnosis and more information, these imaging techniques may be utilized together. At present, there has been reported one-shot investigation methods in which images acquired using different imaging devices for a single lesion area are comparatively investigated for experiments or studies, but there has been proposed no technical means to acquire various kinds of image information simultaneously for the utilization in clinical trials.

### [Disclosure]

### [Technical Problem]

The present disclosure is directed to overcoming the limit of the existing technique in which existing medical imaging devices are individually utilized at diagnosis and medical sites. Also, in the existing technique, due to the absence of a technical measure for simultaneously monitoring a single region in a multilateral way, medical images for an observation area are acquired sporadically depending on a target to be monitored and a purpose of monitoring, and then the images should be analyzed individually by experts. But, the present disclosure is directed to solving such inconvenience.

### [Technical Solution]

In one general aspect, there is provided a device for acquiring an image, comprising: a sound source configured to apply an ultrasound signal for an ultrasound (US) image to a target; a light source configured to apply an optical signal for a photoacoustic (PA) image and a fluorescent (FL) image to the target; a sound probing unit configured to receive the ultrasound signal generated by the sound source and the photoacoustic signal generated by the light source from the target; a light probing unit configured to receive the optical signal generated by the light source from the target; and an image generating unit configured to generate a fusion image including image information with different probing planes with respect to the target by using at least two signals of the received ultrasound signal, the received photoacoustic signal and the received optical signal.

In the device for acquiring an image according to an embodiment, the image generating unit may generate a single fusion image by: generating a depth image of the target from the received ultrasound signal or the received photoacoustic signal, generating a planar image of the target from the received optical signal, and mapping the generated depth image and the generated planar image.

In the device for acquiring an image according to an embodiment, the image generating unit may determine a feature point from each of the images with different probing planes, and map the determined feature points to generate an image where a relation among the images is visually matched and displayed.

In the device for acquiring an image according to an embodiment, the sound probing unit may be located adjacent to the target, and the light probing unit may be installed to be located relatively far from the target in comparison to the sound probing unit.

In the device for acquiring an image according to an embodiment, the sound probing unit and the light probing unit may be installed along different axes to prevent signal interference from each other.

In the device for acquiring an image according to an embodiment, the device may further include a switch for shifting operations of the sound probing unit and the light probing unit to each other, and a signal corresponding to each probing unit may be received according to a manipulation of a user on the switch.

In another aspect of the present disclosure, there is provided a device for acquiring an image, comprising: a sound source configured to apply an ultrasound signal for an ultrasound image to a target; a light source configured to apply an optical signal for a photoacoustic image and a fluorescent image to the target; a sound probing unit configured to receive the ultrasound signal generated by the sound source and the photoacoustic signal generated by the light source from the target; a light probing unit configured to receive the optical signal generated by the light source from the target; a location control unit configured to adjust physical locations of the sound probing unit and the light probing unit; and an image generating unit configured to generate a fusion image including image information with different probing planes with respect to the target by using at least two signals of the received ultrasound signal, the received photoacoustic signal and the received optical signal according to the adjusted locations.

In the device for acquiring an image according to another embodiment, the image generating unit may generate a single fusion image by: generating a three-dimensional image by moving the sound probing unit along a surface of the target according to the control of the location control unit to laminate a depth image of the target from the received ultrasound signal or the received photoacoustic signal, generating a planar image of the target by fixing the location of the light probing unit according to the control of the location control unit, and mapping the generated three-dimensional image and the generated planar image in consideration of the adjusted location.

In the device for acquiring an image according to another embodiment, the location control unit may move the sound probing unit in a longitudinal direction along a surface of the target based on the light probing unit to guide successive generation of depth images corresponding to the planar image by the light probing unit.

In the device for acquiring an image according to another embodiment, the sound probing unit may be located adjacent to the target, the light probing unit may be installed to be located relatively far from the target in comparison to the sound probing unit, and the sound probing unit may receive a sound signal from the target while changing the location thereof according to the control of the location control unit.

In the device for acquiring an image according to another embodiment, the device may further include an optical and/or acoustical transparent front which is adjacent to the target and has permeability with respect to an optical signal and a sound signal.

In another aspect of the present disclosure, there is provided a method for acquiring an image, comprising: applying an ultrasound signal for an ultrasound image or an optical signal for a photoacoustic image to a target, and receiving an ultrasound signal or photoacoustic signal corresponding to a signal applied from the target; applying an optical signal for a fluorescent image to the target, and receiving an optical signal from the target; and generating a fusion image including image information with different probing planes with respect to the target by using at least two signals of the received ultrasound signal, the received photoacoustic signal and the received optical signal, wherein the fusion image includes a depth image generated from the received ultrasound signal or the received photoacoustic signal, a planar image generated from the received optical signal and mapping information between the depth image and the planar image.

In the method for acquiring an image according to an embodiment, the method may further include displaying the generated fusion image on a display device, and the depth image and the planar image included in the fusion image may be shifted to each other according to a manipulation of a user to be displayed simultaneously or in order.

In the method for acquiring an image according to an embodiment, the method may further include generating a three-dimensional image by moving a probing unit for receiving the ultrasound signal or photoacoustic signal in a longitudinal direction along a surface of the target based on the probing unit for the fluorescent image, so that the depth image is successively laminated corresponding to the planar image, and the generating of a fusion image may generate a single fusion image by mapping the generated three-dimensional image and the generated planar image in consideration of the adjusted location.

In the method for acquiring an image according to an embodiment, the method may further include determining a feature point from each of the images with different probing planes, and mapping the determined feature points, and the generating of a fusion image may generate an image in which a relation among the images is visually matched and displayed.

In the method for acquiring an image according to an embodiment, the method may further include: displaying the ultrasound image, the photoacoustic image and the fluorescent image on a display device simultaneously; and generating an overlaying image in which at least two images selected by a user are overlaid, and displaying the overlaying image on the display device.

In the method for acquiring an image according to an embodiment, the method may further include: receiving an adjustment value for a location of the image, a parameter for the image and transparency of the image from the user; and generating an image changed according to the input adjustment value and displaying the changed image on the display device.

### [Advantageous Effects]

The embodiments of the present disclosure allows easier analysis of images by providing a probe structure, which may utilize various medical imaging techniques using an ultrasound image, a photoacoustic image and a fluorescent image simultaneously, provide pathologic information, anatomical information and functional information for a single observation area in a multilateral way by generating a fusion image based on planar image information and depth image information having different probing planes with respect to the observation target, and generate a fusion image through simple user manipulation at a medical site.

### [Description of Drawings]

Fig. 1 is a block diagram showing a basic structure of a device for acquiring an image, employed in embodiments of the present disclosure.
Fig. 2 is a diagram for illustrating structural characteristics of two kinds of probes, employed in embodiments of the present disclosure.
Figs. 3a to 3d are diagrams showing various probe structures, which may be utilized in the device for acquiring an image according to the embodiments of the present disclosure.
Fig. 4 is a diagram showing a fusion image diagnosis system including the device for acquiring an image according to an embodiment of the present disclosure.
Fig. 5 is a flowchart for illustrating a method for acquiring an image according to an embodiment of the present disclosure.
Fig. 6 is a flowchart for illustrating a diagnosis method utilizing an image shifting switch according to an embodiment of the present disclosure.
Fig. 7 is a diagram for illustrating a method for displaying the image acquired according to the diagnosis method of Fig. 6 on a display device.
Fig. 8 is a flowchart for illustrating a diagnosis method utilizing a stationary probe according to another embodiment of the present disclosure.
Fig. 9 is a diagram for illustrating a method for displaying the image acquired according to the diagnosis method of Fig. 8 on a display device.

### [Best Mode]

As an embodiment, the present disclosure provides a device for acquiring an image, which includes: a sound source configured to apply an ultrasound signal for an ultrasound (US) image to a target; a light source configured to apply an optical signal for a photoacoustic (PA) image and a fluorescent (FL) image to the target; a sound probing unit configured to receive the ultrasound signal generated by the sound source and the photoacoustic signal generated by the light source from the target; a light probing unit configured to receive the optical signal generated by the light source from the target; and an image generating unit configured to generate a fusion image including image information with different probing planes with respect to the target by using at least two signals of the received ultrasound signal, the received photoacoustic signal and the received optical signal.

### [Mode for Invention]

Hereinafter, a basic idea adopted in embodiments of the present disclosure will be described briefly, and then detailed technical features will be described in order.

A biological tissue causes a radiative process and a nonradiative process through a photoacoustic coefficient, and a fluorescent image and a photoacoustic image are formed by means of different process bases through absorbed optical energy. The embodiments of the present disclosure allow observing the degree of light absorption and the generation of radiative/nonradiative process of a tissue, thereby proposing a system structure which may obtain an optical characteristic of the tissue as a more accurate quantitative index and provide elastic ultrasound image (elastography) and color flow imaging by processing the ultrasound signal. In addition, the embodiments of the present disclosure may provide a quantitative index with high contrast in an application using a contrast agent which is reactive with a single imaging technique or multiple imaging techniques, and propose individual information for various imaging techniques or applications used in existing ultrasound, photoacoustic and fluorescent imaging, or a structure required for developing a new application by combining such individual information.

For this, there is required a fusion probe and system structure capable of performing ultrasound, photoacoustic and fluorescent imaging techniques to a single tissue and processing them in association with each other in a bundle. In addition, there is proposed an auxiliary system structure for improving the shape of the fusion probe, the structure of the system and the quality of the image.

Hereinafter, embodiments of the present disclosure which may be easily implemented by those skilled in the art will be described in detail. However, these embodiments are just for better understanding of the present disclosure, and it is obvious to those skilled in the art that the scope of the present disclosure is not limited thereto.

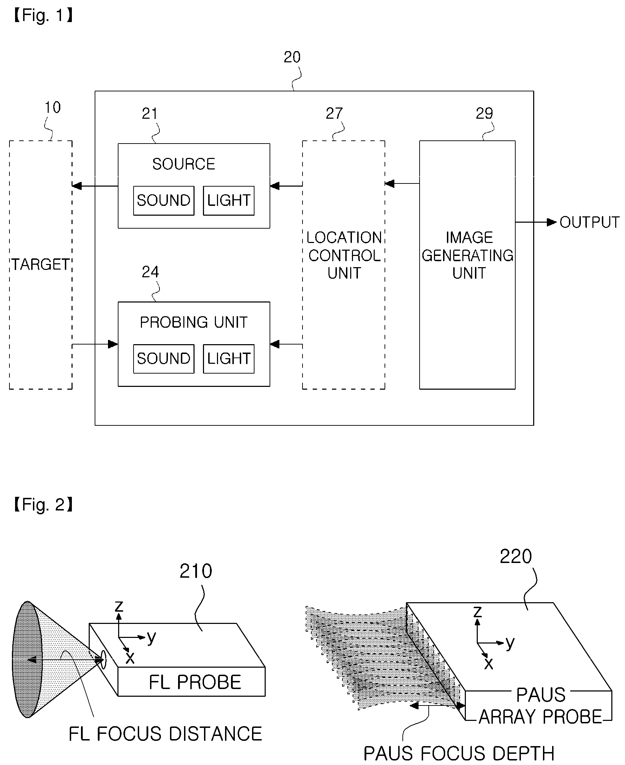

Fig. 1 is a block diagram showing a basic structure of a device 20 for acquiring an image (hereinafter, also referred to as an image acquiring device), employed in embodiments of the present disclosure, and the image acquiring device 20 includes a source 21 and a probing unit 24 used adjacent to an observation target 10, and an image generating unit 29 respectively electrically connected thereto.

The source 21 may be classified into a sound source and a light source depending on the kind of generated signal. The sound source applies an ultrasound signal for an ultrasound (US) image to a target 10, and the light source applies an optical signal for a photoacoustic (PA) image and a fluorescent (FL) image to the target 10.

In addition, the probing unit 24 may be classified into a sound probing unit and a light probing unit depending on the kind of received signal. The sound probing unit receives an ultrasound signal generated by the sound source or a photoacoustic signal generated by the light source from the target 10, and the light probing unit receives an optical signal generated by the light source from the target 10.

In Fig. 1, the source 21 and the probing unit 24 are depicted separately in a functional view, but they may also be implemented as a physically integrated unit. In particular, depending on the kind of image to be obtained from the target 10, the source 21 and the probing unit 24 may be implemented to be included in the same component. For example, the ultrasound image and the photoacoustic image may be implemented in the same hardware since a carrier signal to be probed from the target 10 is an ultrasound signal. However, a detailed source of the photoacoustic image is a pulsed wave, different from the source of the ultrasound image. Meanwhile, the fluorescent image applies an optical signal, in more detail a continuous wave and receives an optical signal emitted from a tissue of the target 10 by using a CCD camera, which allows implementation in single hardware.

Further, the sound probing unit and the light probing unit have different minimum distances to the observation target 10 depending on signal observation characteristics. In other words, the sound probing unit may be located adjacent to the observation target 10, and the light probing unit may be installed to be located relatively far from the observation target 10 in comparison to the sound probing unit. This is because a probe based on a sound signal like the ultrasound image is used in contact with the observation target 10, and a probe based on an optical signal like the fluorescent image is used at a predetermined distance to observe a planar structure like the surface of the observation target 10.

The image generating unit 29 generates a fusion image including image information with different probing planes with respect to the target 10 by using at least two signals of the ultrasound signal, the photoacoustic signal and the optical signal received from the probing unit 24. In the embodiments of the present disclosure, a single fusion image including various kinds of information may be generated by collecting a plurality of image information with different probing planes, and the detailed configuration of each embodiment will be described later with reference to the drawings.

In addition, the image acquiring device of Fig. 1 may selectively include a location control unit 27 between the source 21 and/or the probing unit 24 and the image generating unit 29. The location control unit 27 adjusts physical locations of the sound probing unit and the light probing unit and guides the image generating unit 29 to generate a fusion image from the signals received according to the locations adjusted by the location control unit 27.

In particular, the image generating unit 29 moves the probing unit 24, particularly the sound probing unit, along the surface of the target 10 according to the control of the location control unit 27, so that a depth image of the target 10 is laminated from the received ultrasound signal or photoacoustic signal to generate a three-dimensional image. Further, the image generating unit 29 may generate a planar image of the target 10 by fixing the location of the probing unit 24, particularly the light probing unit, according to the control of the location control unit 27, and may generate a single fusion image by mapping the three-dimensional image and the planar image generated in consideration of a finally adjusted location. In other words, the location control unit 27 is required for generating a three-dimensional image from the depth image by controlling the location of the probing unit 24.

Or else, instead of the control of the location control unit 27, a user may directly moves the probing unit 24 along the surface of the target 10 so that the light probing unit is located at the same portion as the acquired ultrasound signal or photoacoustic signal to acquire a fluorescent planar image, thereby providing a single fusion image by means of software image mapping.

Data of each imaging technique used in the above image mapping procedure may employ a high-contrast imaging method, used in individual techniques, to improve the quality of image. The ultrasound image may utilize, for example, harmonic, perfusion imaging, synthetic aperture, planar wave, blood flow imaging, adaptive beam focusing or the like. In addition, the photoacoustic image may utilize, for example, adaptive beam focusing, spectroscopy or the like. Further, the fluorescent image may utilize, for example, stereo 3D imaging, spectroscopy, wavelength separating or the like.

Meanwhile, the image generating unit 29 may determine a feature point from each of images with different probing planes and map the determined feature point, thereby generating an image where a relation of the images may be visually matched and displayed. For this, an image processing technique for extracting feature points from a plurality of images and mapping the feature points may be utilized. When mapping images, basically, an axis of one image is specified based on the target 10, and images are connected on the basis of the specified axis, so that a relation of common features is displayed. For this, a three-dimensional coordinate system for the target 10 is assumed, a display direction of the image with respect to x-, y- and z-directions of the coordinate system is set, and the images are mapped based on the feature point to generate a matched image.

Hereinafter, characteristics of an individual probe according to a medical imaging technique will be introduced briefly, and then various probe structures mechanically coupled to generate a fusion image will be described in order.

Fig. 2 is a diagram for illustrating structural characteristics of two kinds of probes, employed in embodiments of the present disclosure, where a fluorescent image probe (FL probe) 210 for generating a planar image of a target and an photoacoustic/ultrasound image array probe (PAUS array probe) 220 for generating a depth image of a target are depicted. In a hardware aspect, each probe may be implemented to include a signal applying unit (source) and a signal receiving unit as a single unit, as described above.

Characteristics of each medical image are shown in Table 1 below.

**[Table 1]**

| | Source | Probing unit | Description |
|---|---|---|---|
| Ultrasound image | Ultrasound signal | Transducer | Provision of anatomical information based on a depth image |
| Photoacousti c image | Optical signal /pulsed wave | Transducer | Provision of functional information based on a depth image |
| Fluorescent image | Optical signal /continuous wave | CCD sensor | Provision of functional information based on a planar image |

In Fig. 2, the FL probe 210 includes a light applying unit capable of applying light and a CCD sensor capable of receiving a fluorescent signal generated from a target tissue to acquire a fluorescent (FL) image and a white light (WL) image, and the PAUS array probe 220 includes a light applying unit capable of transmitting a pulsed laser and an array transducer capable of receiving an ultrasound to acquire data of a photoacoustic image and an ultrasound image. The FL light applying unit and the PAUS light applying unit may be designed to be integrated into a single component or separated from each other.

Considering the above different characteristics, the image generating unit proposed in the embodiments of the present disclosure generates a depth image of the target from the ultrasound signal or photoacoustic signal received through the sound probing unit, generate a planar image of the target from the optical signal received through the light probing unit, and maps the generated depth image and the generated planar image to generate a single fusion image.

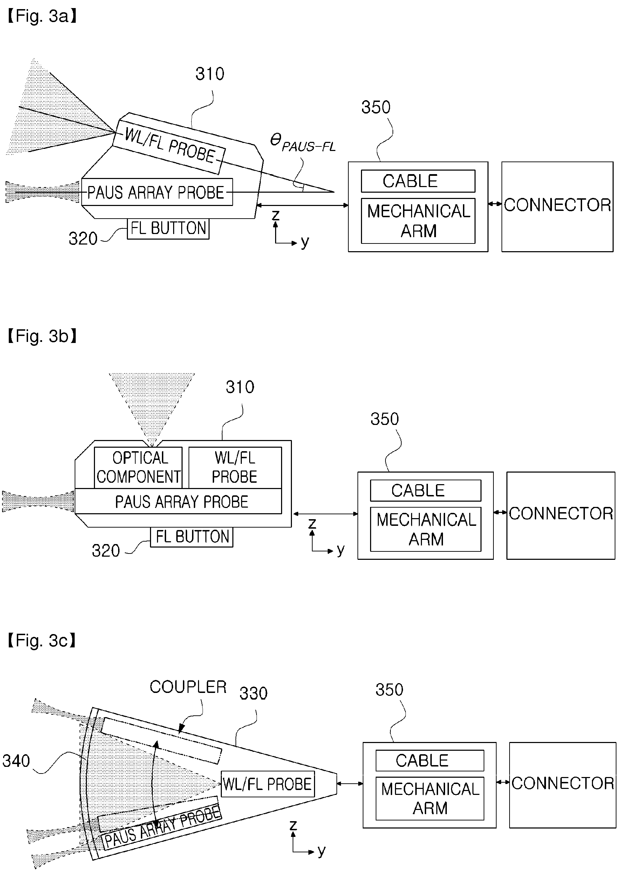

Figs. 3a to 3d are diagrams showing various probe structures, which may be utilized in the image acquiring device according to the embodiments of the present disclosure, and these structures may be utilized in two ways briefly..

First, Figs. 3a and 3b show a probe structure for real-time PAUS and FL imaging, where the PAUS probe and the FL probe are implemented to be fixed in a single probe structure 310, and a PAUS image and a FL image may be output alternately according to a manipulation (PUSH/RELEASE) of the FL button 320 which corresponds to a switch for shifting operations of the sound probing unit and the light probing unit to each other. Now, the image acquired through the probe structure 310 may be provided to an imaging system through a connector via a connection unit 350 having a cable. If necessary, the connection unit 350 may further include a mechanical arm for operating the probe structure 310 at a position closer to the observation target.

In addition, in Figs. 3a and 3b, the sound probing unit (PAUS array probe) and the light probing unit (WL/FL probe) may be installed along different axes to prevent signal interference from each other.

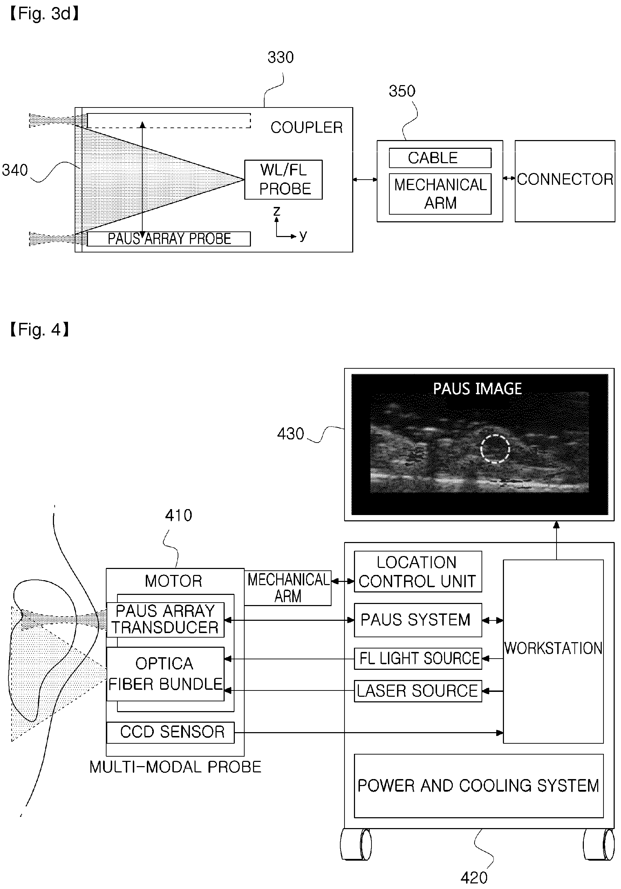

Second, Figs. 3c and 3d show a fusion probe structure capable of displaying a PAUS image and a FL image at the same location of a human body in a fused state, and the structure includes a movable PAUS probe and a stationary WL/FL probe in a single probe structure 330. The PAUS probe moves vertically by means of a mechanically movable scanner to acquire data of a three-dimensional image, and the FL probe is located at a rear position of the fusion probe to acquire a planar image of the image region. For this, the image acquiring device proposed in the embodiments of the present disclosure may move the sound probing unit (PAUS array probe) in a longitudinal direction along the surface of the target based on the light probing unit (WL/FL probe) by using the location control unit (not shown), thereby guiding a depth image to be successively generated corresponding to the planar image by the light probing unit (WL/FL probe).

In addition, due to the difference in image acquiring structures as described above, the sound probing unit (PAUS array probe) may be located adjacent to the target, the light probing unit (WL/FL probe) may be installed to be located relatively far from the target in comparison to the sound probing unit (PAUS array probe), and the sound probing unit (PAUS array probe) may receive a sound signal from the target while changing its location according to the control of the location control unit (not shown).

Further, the inside of the probe may be filled with a coupler capable of transmitting light without loss and allowing ultrasound permeation, and the surface of the probe may be made of a material allowing permeation of ultrasound and light. For this, the probe structure 330 depicted in Figs. 3c and 3d may include an optical/acoustical transparent front 340 which is adjacent to the target and has permeability with respect to optical signals and sound signals.

Fig. 4 is a diagram showing a fusion image diagnosis system including the image acquiring device according to an embodiment of the present disclosure, and the fusion image diagnosis system includes a multi-modal probe 410 having a plurality of sources and probes, an image processing system 420 and a display device 430.

The image processing system 420 includes a workstation for controlling the overall fusion image diagnosis system, a PAUS system for treating signals of photoacoustic and ultrasound images, a FL light source for applying an optical energy for the fluorescent image and the photoacoustic image, and a probe location control unit (probe positioner) capable of controlling a location of the probe as demanded by the user, and acquires bio data in various aspects through the multi-modal probe 410 serving as a fusion probe.

The multi-modal probe 410 includes a PAUS linear transducer for receiving photoacoustic and ultrasound signals, an optic fiber bundle for applying an optical energy transmitted from a main body, and a CCD sensor for acquiring fluorescent data generated from the human body, and transmits the acquired data to the image processing system 420. The image processing system 420 performs image restoration based on the received data and then displays the restored image on the display device 430.

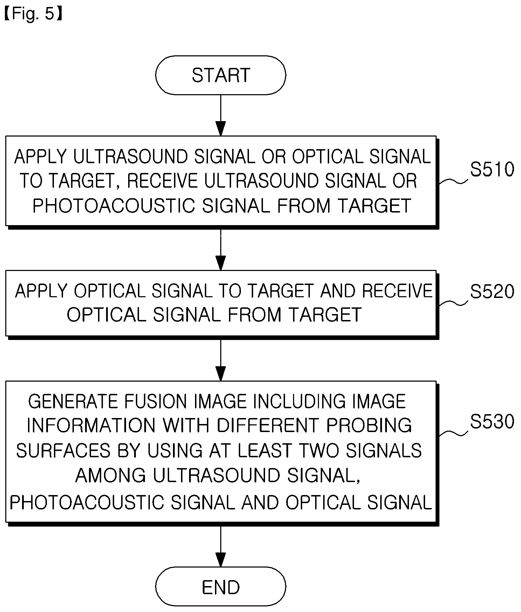

Fig. 5 is a flowchart for illustrating a method for acquiring an image (hereinafter, also referred to as an image acquiring method) according to an embodiment of the present disclosure, which includes steps respectively corresponding to operations of the components of the image acquiring device depicted in Fig. 1. Therefore, each process will be briefly described based on the image processing flow in order to avoid unnecessary duplicated explanations.

In S510, the image acquiring device applies an ultrasound signal for the ultrasound image and an optical signal for the photoacoustic image to the target, and receives an ultrasound signal or photoacoustic signal corresponding to the signal applied from the target.

In S520, the image acquiring device applies an optical signal for the fluorescent image to the target, and receives an optical signal from the target.

In S530, the image acquiring device generates a fusion image including image information with different probing planes for the target by using at least two signals among the ultrasound signal and the photoacoustic signal received in S510 and the optical signal received in S520. Here, the fusion image may include a depth image generated from the ultrasound signal or photoacoustic signal, a planar image generated from the optical signal, and mapping information between the depth image and the planar image.

Meanwhile, the image acquiring method as depicted in Fig. 5 may further include a step of determining a feature point from each of images with different probing planes, mapping the determined feature points. Therefore, in the fusion image generating step S530, an image where a relation between images is visually matched and displayed may be generated.

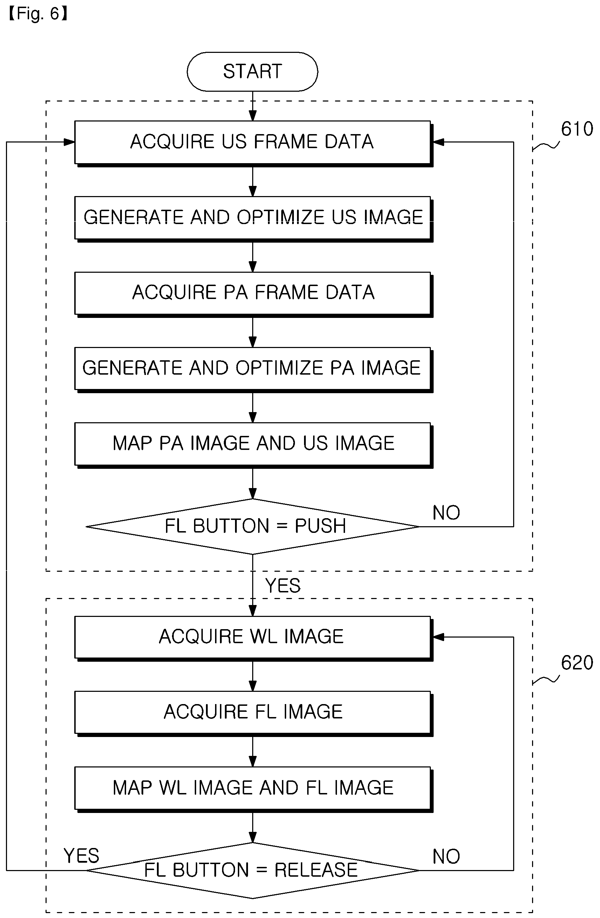

Fig. 6 is a flowchart for illustrating a diagnosis method utilizing an image shifting switch according to an embodiment of the present disclosure, which is briefly classified into an image acquiring process 610 based on a sound signal and an image acquiring process 620 based on an optical signal.

Fig. 6 assumes a surgery mode in which a user freely utilizes the probe structure to approach or contact a part of a body of a patient and acquires a necessary image, and a sequence of operations utilizable for subsequently acquiring a PAUS image and a FL image by using the probe structure 310 of Fig. 3a or 3b is exemplarily depicted. For this, the image acquiring method according to an embodiment of the present disclosure may further include a process of displaying the generated fusion image on the display device, and here, an input unit for switching the depth image and the planar image included in the fusion image according to a manipulation of the user to be displayed simultaneously or subsequently may be used.

Referring to Fig. 6, when an initial system operation is performed, the PAUS image acquired through the probe structure is provided, and if the user pushes the FL button, the operation may be shifted to a FL image display mode so that an image where a white light (WL) image is fused with a fluorescent (FL) image is provided to the user. A process of shifting to a PAUS mode by pressing a PAUS button or a process of shifting an image mode whenever a switch button is pushed may also be implemented.

In more detail, the sound signal-based image acquiring process 610 firstly acquires US frame data and generates and optimizes a US image therefrom, and also acquires PA frame data and generates and optimizes a PA image therefrom. After that, the generated PA image and US image may be mapped to generate image information in a depth direction. Now, the user presses the FL button to shift into another fluorescent image mode.

The optical signal-based image acquiring process 620 may firstly acquire a WL image and a FL image respectively, and map a WL image and a FL image therefrom to generate image information in a front direction. Now, if the user releases the FL button, the process returns to the PAUS image acquiring process 610.

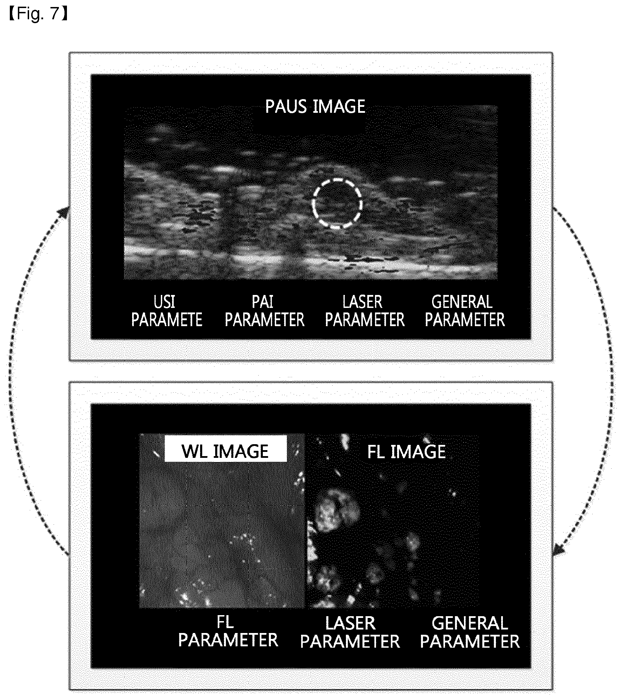

Fig. 7 is a diagram for illustrating a method for displaying the image acquired according to the diagnosis method of Fig. 6 on a display device, and an utilizable graphic user interface (GUI) is proposed.

Referring to Fig. 7, by manipulating the switch, the PAUS image and the FL image may be alternately provided to the user in real time, and restoration information or image parameter may be set for each image mode. A parameter setting unit may be provided simultaneously or separately for the PAUS image and the FL image. In addition, by simultaneously displaying a white light image and a fluorescent image in parallel or in an overlaid state, characteristics of several images may be utilized together for diagnosis.

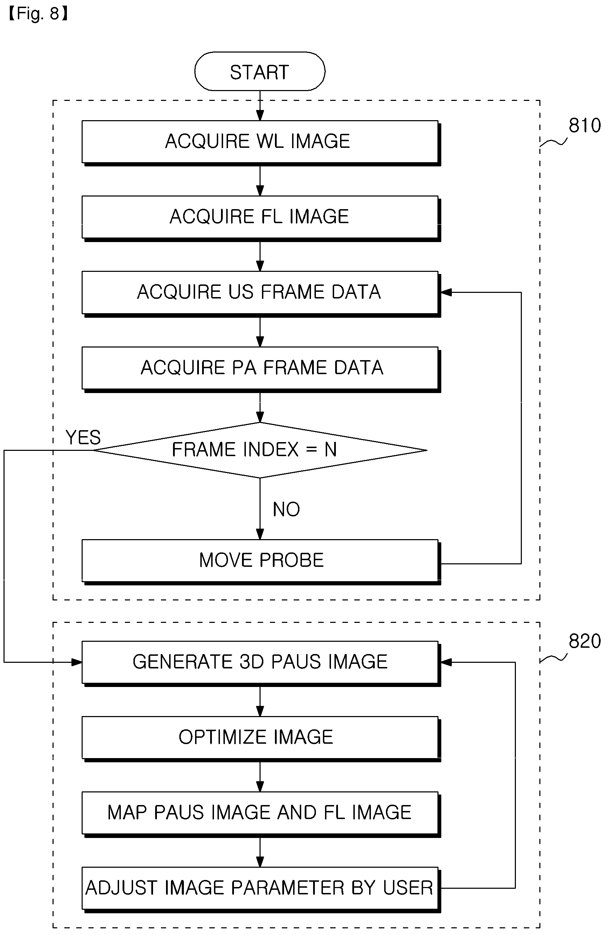

Fig. 8 is a flowchart for illustrating a diagnosis method utilizing a stationary probe according to another embodiment of the present disclosure, which is briefly classified into an individual image acquiring process 810 and a three-dimensional image generating process 820. Here, a stationary probe means a probe structure which may be fixed to an observation target and acquire a three-dimensional image, and different from a fixed probe structure, the probe for acquiring PA/US images provided in the probe structure obtains a three-dimensional image through movement, paradoxically.

In Fig. 8, a user does not freely utilize the probe structure, but an image registration mode in which the probe structure is fixed in contact with or adjacent to a part of a body of a patient and then acquires various types of images for a single observation target is assumed, and also a sequence of operations utilizable for obtaining the PAUS image and the FL image by using the probe structure 330 of Fig. 3c or 3d is exemplarily depicted.

For this, the image acquiring device available for this embodiment moves the probing unit (PAUS array probe) for receiving an ultrasound signal or a photoacoustic signal in a longitudinal direction along the surface of the observation target on the basis of the probing unit (FL probe) for a fluorescent image, so that a depth image is successively laminated corresponding to the planar image to generate a three-dimensional image. At this time, in the fusion image generating process, the three-dimensional image and the planar image are mapped in consideration of the location adjusted through the location control unit to generate a single fusion image.

First, in the individual image acquiring process 810, a WL image and a FL image are acquired, US frame data and PA frame data are acquired along with the images, and then a PAUS image of a single frame is acquired. Now, the PAUS array probe is moved in a longitudinal direction of the observation target to acquire a PAUS image of a neighboring frame. This depth image acquiring process for each frame is repeatedly performed until a desired number of frames (for example, until an index of a final frame reaches a preset positive integer N), and then the individual image acquiring process 810 is completed. At this time, the generated image includes a single surface image and N number of depth images for the interested area.

Now, in the three-dimensional image generating process 820, the N number of depth images generated in the longitudinal direction is laminated to generate a single three-dimensional PAUS image. After that, the generated PAUS image is optimized, and then the PAUS image and the FL image are mapped and displayed on a single display device. The user may reconfigure the displayed image by adjusting and resetting image parameters as necessary.

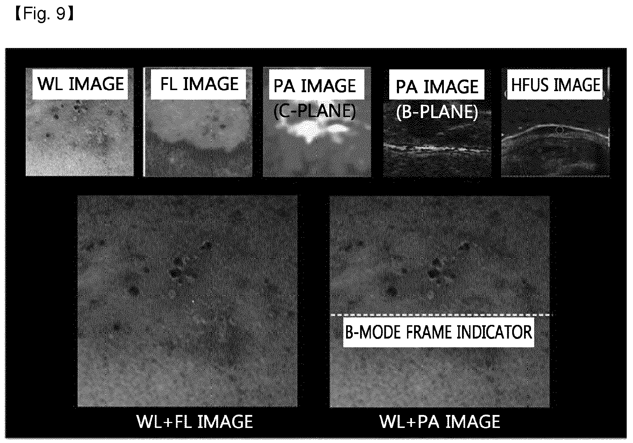

Fig. 9 is a diagram for illustrating a method for displaying the image acquired according to the diagnosis method of Fig. 8 on a display device, and an utilizable graphic user interface (GUI) is proposed.

Referring to Fig. 9, images may be restored according to a plurality of image modes and provided simultaneously, and thus it is possible to provide the maximum degree of freedom as required by the user on the basis of various image data acquirable through the probe structure. The image acquired through the probe structure may provide a WL image, a FL image, a PA image (C-plane, B-plane), a US image (B-mode, elastography, color) or the like, depending on the selection of the user before the image is acquired. In Fig. 9, the user may acquire human biometric information by freely adjusting an adjustment value for a location of the image, a parameter for the image and transparency of the individual image to be fused, by using the data of the PAUS image, the WL image and the FL image acquired through the probe structure.

For this, in the image acquiring method according to the present disclosure, the ultrasound image, the photoacoustic image and the fluorescent image may be simultaneously displayed on the display device, and an image in which at least two images selected by the user are overlaid may be generated and displayed on the display device. In addition, in the image acquiring method according to the present disclosure, an adjustment value for a location of the image, a parameter for the image and transparency of the image may be input by the user, and an image changed according to the input adjustment value may be generated and displayed on the display device.

According to the embodiments of the present disclosure described above, at preclinical trials, delivery of medicine and resultant effects may be quantitatively figured out by means of reaction against light, thereby allowing more quantitative evaluation of medicine effects. In addition, at clinical trials, light characteristics of tissues having different clinical meanings may be more quantitatively understood to allow early diagnosis of diseases and accurate staging, which may be helpful for establishing a plane for treating a disease and actually treating the disease.

Further, the embodiments of the present disclosure may be applied in various ways by combining advantages of a photoacoustic/ultrasound imaging technique capable of observing a relatively greater depth for an observation target and a fluorescent imaging technique capable of observing an overall surface at a relatively smaller depth. In addition, if a contrast agent is used, characteristics of materials in or out of the contrast agent may be differently set for photoacoustic and fluorescent images, and then the distribution of the contrast agent and the degree of transfer of medicine included in the contrast agent may be quantitatively figured out.

Meanwhile, a motion control process of a probe structure and an image processing process for processing individual images obtained by the probe structure according to the embodiment of the present disclosure may be implemented as computer-readable codes on a computer-readable recording medium. The computer-readable recording medium may include any kind of storage devices where data readable by a computer system is stored.

The computer-readable recording medium includes, for example, ROM, RAM, CD-ROM, a magnetic tape, a floppy disk and optical media, and may also be implemented in the form of carrier wave (for example, transmission through Internet). In addition, the computer-readable recording medium may be distributed to computer systems connected through a network so that computer-readable codes may be stored and executed in a distribution way. Also, functional programs, codes and code segments for implementing the present disclosure may be easily inferred by programmers in the related art.

While the exemplary embodiments have been shown and described, it will be understood by those skilled in the art that various changes in form and details may be made thereto without departing from the spirit and scope of this disclosure as defined by the appended claims. In addition, many modifications can be made to adapt a particular situation or material to the teachings of this disclosure without departing from the essential scope thereof. Therefore, the spirit of the present disclosure should not be limited to the embodiments described above, and the appended claims and their equivalents or modifications should also be regarded as falling within the scope of the present disclosure.

### [Industrial Applicability]

According to the embodiments of the present disclosure, there is provided a probe structure, which may utilize various medical imaging techniques such as ultrasound imaging, photoacoustic imaging and fluorescent imaging simultaneously, generate a fusion image based on planar image information and depth image information with different probing planes with respect to an observation target so that pathologic information, anatomical information and functional information may be provided in a multilateral way with respect to a single observation area, and allow a user to generate a fusion image in a medical site just through a simple manipulation, thereby allowing easier image analysis.

## Claims

1. A device for acquiring an image, comprising:
a sound source configured to apply an ultrasound signal for an ultrasound (US) image to a target;
a light source configured to apply an optical signal for a photoacoustic (PA) image and a fluorescent (FL) image to the target;
a sound probing unit configured to receive the ultrasound signal generated by the sound source and the photoacoustic signal generated by the light source from the target;
a light probing unit configured to receive the optical signal generated by the light source from the target; and
an image generating unit configured to generate a fusion image including image information with different probing planes with respect to the target by using at least two signals of the received ultrasound signal, the received photoacoustic signal and the received optical signal.

2. The device for acquiring an image according to claim 1,
wherein the image generating unit generates a single fusion image by:
generating a depth image of the target from the received ultrasound signal or the received photoacoustic signal,
generating a planar image of the target from the received optical signal,
and
mapping the generated depth image and the generated planar image.

3. The device for acquiring an image according to claim 1,
wherein the image generating unit determines a feature point from each of the images with different probing planes, and maps the determined feature points to generate an image where a relation among the images is visually matched and displayed.

4. The device for acquiring an image according to claim 1,
wherein the sound probing unit is located adjacent to the target, and wherein the light probing unit is installed to be located relatively far from the target in comparison to the sound probing unit.

5. The device for acquiring an image according to claim 1,
wherein the sound probing unit and the light probing unit are installed along different axes to prevent signal interference from each other.

6. The device for acquiring an image according to claim 1, further comprising:
a switch for shifting operations of the sound probing unit and the light probing unit to each other,
wherein a signal corresponding to each probing unit is received according to a manipulation of a user on the switch.

7. A device for acquiring an image, comprising:
a sound source configured to apply an ultrasound signal for an ultrasound image to a target;
a light source configured to apply an optical signal for a photoacoustic image and a fluorescent image to the target;
a sound probing unit configured to receive the ultrasound signal generated by the sound source and the photoacoustic signal generated by the light source from the target;
a light probing unit configured to receive the optical signal generated by the light source from the target;
a location control unit configured to adjust physical locations of the sound probing unit and the light probing unit; and
an image generating unit configured to generate a fusion image including image information with different probing planes with respect to the target by using at least two signals of the received ultrasound signal, the received photoacoustic signal and the received optical signal according to the adjusted locations.

8. The device for acquiring an image according to claim 7,
wherein the image generating unit generates a single fusion image by:
generating a three-dimensional image by moving the sound probing unit along a surface of the target according to the control of the location control unit to laminate a depth image of the target from the received ultrasound signal or the received photoacoustic signal,
generating a planar image of the target by fixing the location of the light probing unit according to the control of the location control unit, and
mapping the generated three-dimensional image and the generated planar image in consideration of the adjusted location.

9. The device for acquiring an image according to claim 7,
wherein the location control unit moves the sound probing unit in a longitudinal direction along a surface of the target based on the light probing unit to guide successive generation of depth images corresponding to the planar image by the light probing unit.

10. The device for acquiring an image according to claim 7,
wherein the sound probing unit is located adjacent to the target,
wherein the light probing unit is installed to be located relatively far from the target in comparison to the sound probing unit, and
wherein the sound probing unit receives a sound signal from the target while changing the location thereof according to the control of the location control unit.

11. The device for acquiring an image according to claim 7, further comprising:
an optical and/or acoustical transparent front which is adjacent to the target and has permeability with respect to an optical signal and a sound signal.

12. A method for acquiring an image, comprising:
applying an ultrasound signal for an ultrasound image or an optical signal for a photoacoustic image to a target, and receiving an ultrasound signal or photoacoustic signal corresponding to a signal applied from the target;
applying an optical signal for a fluorescent image to the target, and receiving an optical signal from the target; and
generating a fusion image including image information with different probing planes with respect to the target by using at least two signals of the received ultrasound signal, the received photoacoustic signal and the received optical signal,
wherein the fusion image includes a depth image generated from the received ultrasound signal or the received photoacoustic signal, a planar image generated from the received optical signal and mapping information between the depth image and the planar image.

13. The method for acquiring an image according to claim 12, further comprising:
displaying the generated fusion image on a display device,
wherein the depth image and the planar image included in the fusion image are shifted to each other according to a manipulation of a user to be displayed simultaneously or in order.

14. The method for acquiring an image according to claim 12, further comprising:
generating a three-dimensional image by moving a probing unit for receiving the ultrasound signal or photoacoustic signal in a longitudinal direction along a surface of the target based on the probing unit for the fluorescent image, so that the depth image is successively laminated corresponding to the planar image,
wherein the generating of a fusion image generates a single fusion image by mapping the generated three-dimensional image and the generated planar image in consideration of the adjusted location.

15. The method for acquiring an image according to claim 12, further comprising:
determining a feature point from each of the images with different probing planes, and mapping the determined feature points,
wherein the generating of a fusion image generates an image in which a relation among the images is visually matched and displayed.

16. The method for acquiring an image according to claim 12, further comprising:
displaying the ultrasound image, the photoacoustic image and the fluorescent image on a display device simultaneously; and
generating an overlaying image in which at least two images selected by a user are overlaid, and displaying the overlaying image on the display device.

17. The method for acquiring an image according to claim 12, further comprising:
receiving an adjustment value for a location of the image, a parameter for the image and transparency of the image from the user; and
generating an image changed according to the input adjustment value and displaying the changed image on the display device.
